(19) Europäisches Patentamt
European Patent Office
Office européen des brevets

(11) EP 2 559 456 B1

(12) **EUROPÄISCHE PATENTSCHRIFT**

(45) Veröffentlichungstag und Bekanntmachung des Hinweises auf die Patenterteilung:
**05.09.2018 Patentblatt 2018/36**

(51) Int Cl.:
***A61Q 5/02*** *(2006.01)* ***A61Q 5/10*** *(2006.01)*
***A61K 8/898*** *(2006.01)* ***A61K 8/41*** *(2006.01)*

(21) Anmeldenummer: **12175289.3**

(22) Anmeldetag: **06.07.2012**

(54) **Mittel zum Behandeln und/oder Färben keratinischer Fasern, enthaltend spezifische Aminosilikone und Alkalisierungsmittel**

Agent for treating and/or dying keratin fibres containing specific aminosilicones and alkalising agent

Agent de traitement et/ou de coloration des fibres kératiniques, contenant des aminosilicones spécifiques et agents alcalinisants

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.08.2011 DE 102011081190**

(43) Veröffentlichungstag der Anmeldung:
**20.02.2013 Patentblatt 2013/08**

(73) Patentinhaber: **Henkel AG & Co. KGaA**
**40589 Düsseldorf (DE)**

(72) Erfinder:
- **Goutsis, Konstantin**
**41363 Jüchen (DE)**
- **Weser, Gabriele**
**41472 Neuss (DE)**

(56) Entgegenhaltungen:
**EP-A1- 1 559 409**
**WO-A1-2010/140544**
**WO-A2-2012/079915**
**US-A- 4 820 308**
**US-A1- 2003 147 841**

- **MOMENTIVE: "Silsoft AX", INTERNET CITATION, 1. März 2010 (2010-03-01), Seiten 1-12, XP002675429, Gefunden im Internet: URL:http://www.silicones.com/momentiveInternetDoc/Internet/Static%20Files/Documents/Marketing%20Bulletin/Silsoft%20AX%20MB%20i ndd.pdf [gefunden am 2012-05-08]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 559 456 B1

## Beschreibung

Die vorliegende Erfindung betrifft Mittel zum Behandeln von Haaren, insbesondere menschlicher Haare, die mindestens ein spezifisches Aminosilikon und mindestens ein Alkalisierungsmittel enthalten. Weiterhin betrifft die vorliegende Erfindung die Verwendung dieser Mittel zum Färben und/oder Aufhellen von Haaren sowie ein entsprechendes Verfahren.

Die Pflege und Behandlung des menschlichen Haares stellt einen wichtigen Bestandteil der täglichen Hygiene dar, und auch die Veränderung von Form und Farbe der Haare ist ein wichtiger Bereich der modernen Kosmetik. Die Behandlung mit Shampoos und Conditionern zählt zu den üblicherweise mehrmals die Woche, oft auch täglich angewandten Haarbehandlungsmitteln. Veränderungen von Form und Farbe der Haare erfolgen üblicherweise in größeren Zeitabständen, sind für den Verbraucher aber im Hinblick auf das eigene Erscheinungsbild von ähnlich großer Bedeutung. Insbesondere die Veränderung der eigenen Haarfarbe ist für den Verbraucher sowohl eine modische Ausdrucksform als auch eine Möglichkeit zur Kaschierung von ergrauten Haaren.

Zur modischen Farbgestaltung von Frisuren oder zur Kaschierung von ergrautem oder gar weißem Haar mit modischen oder natürlichen Farbtönen greift der Verbraucher zu farbverändernden Mitteln.

Zur Bereitstellung farbverändernder kosmetischer Mittel, insbesondere für die Haut oder keratinhaltige Fasern wie beispielsweise menschliche Haare, kennt der Fachmann je nach Anforderungen an die Färbung bzw. Farbveränderung diverse Systeme.

Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich durch intensive hervorragende, lang anhaltende Färbeergebnisse aus. Für natürlich wirkende Färbungen kann eine Mischung aus einer größeren Zahl von Oxidationsfarbstoffvorprodukten eingesetzt werden, wobei in vielen Fällen weiterhin zusätzlich direktziehende Farbstoffe zur Nuancierung verwendet werden.

Sollen Keratinfasern aufgehellt oder gar gebleicht werden, werden die das Substrat färbenden Farbstoffe meist oxidativ unter Einsatz von entsprechenden Oxidationsmitteln, wie beispielsweise Wasserstoffperoxid, entfärbt.

Trotz bereits vorhandener, hoch entwickelter Färbesysteme besteht weiterhin Bedarf an Färbesystemen, die eine hervorragende Leuchtkraft und Intensität der Färbungen erreichen, gleichzeitig jedoch über eine sehr gute Haltbarkeit und eine hervorragende Homogenität verfügen. Die Färbesysteme sollen sich neben guten Echtheitseigenschaften auch durch ein gutes anwendungstechnisches Profil auszeichnen, sie sollen ausreichend lagerstabil sein, eine gute Anmischbarkeit besitzen und im Hinblick auf ihre Viskosität optimal eingestellt sein.

Bedingt durch die im Oxidationsfärbemittel enthaltenen Oxidationsmittel werden nicht nur die für die Färbung verantwortlichen Oxidationsfarbstoffvorprodukte oxidiert, sondern auch die Strukturbestand-teile des Haares selbst oxidativ verändert. Zusätzlich führt der üblicherweise in solchen Mitteln basisch eingestellte pH-Wert dazu, dass die Struktur des Haares oxidativ geschädigt wird. Je nach Ausprägung des Schädigungsgrades reicht dieser von rauem, sprödem und schwieriger auskämmbarem Haar über eine verminderte Widerstandsfähigkeit und Reißfestigkeit des Haares bis hin zu Haarbruch. Je größer die Menge des eingesetzten Wasserstoffperoxids ist, desto stärkere Schädigungen werden in der Regel auf der Keratinfaser hervorgerufen. Insbesondere Spliss oder gar Bruch der Faser sind vom Verbraucher höchst unerwünschte Begleiterscheinungen einer oxidativen Haarbehandlung, die insbesondere bei mehrfacher Wiederholung der oxidativen Haarbehandlung auftreten können.

Es ist daher besonders wünschenswert, Färbemittel bereitzustellen, die neben einer guten Färbeleistung einen positiven Beitrag zur Verbesserung der Faserstruktur leisten und damit gleichzeitig als Pflege- oder Konditioniermittel wirken. Somit könnte auf die sonst häufig erforderliche, zusätzliche pflegende Nachbehandlung verzichtet werden, die sowohl hinsichtlich des Anwendungskomforts als auch im Hinblick auf die Verpackungsökonomie mit Nachteilen behaftet ist.

Dem Marktsegment der Haarcolorationen für Männer kommt eine stetig wachsende Bedeutung zu. Männer stellen jedoch spezielle Anforderungen Colorationsprodukte, die von den Anforderungen, die ein typisches, im Markt befindliches Colorationsprodukt für Frauen erfüllt, abweichen. Männer färben ihre Haare hauptsächlich zur Abdeckung von ergrauten Haaren und bevorzugen unauffälligere Naturnuancen. Wichtig ist hierbei vor allem ein Farbergebnis, das mit der ursprünglichen Haarfarbe möglichst deckungsgleich übereinstimmt und nicht auf eine offensichtliche kosmetische Behandlung hinweist. Modische Nuancen liegen weniger im Fokus, und insbesondere ein stark glänzendes Farbergebnis ist unerwünscht.

Es ist die Aufgabe der vorliegenden Erfindung, Haarbehandlungsmittel bereitzustellen, welche die zuvor aufgezählten Nachteile nicht besitzen. Haarfärbemittel sollen intensive Färbungen mit hoher Leuchtkraft erzeugen, stabil und komfortabel applizierbar sein. Weiterhin sollen die Färbemittel pflegende und strukturierende Eigenschaften besitzen, weder zu Augen- noch zu Kopfhautreizungen führen, die Haare glätten und deren Kämmbarkeit verbessern. Ein

Hauptaugenmerk der Aufgabenstellung liegt darüber hinaus auf der Bereitstellung eines auch im Marktssegment der Männercolorationen einsetzbaren Mittels, welches natürliche Nuancen ohne starken Glanzeffekt liefert.

In überraschender Weise konnte nun gefunden werden, dass Mittel zum Behandeln von keratinischen Fasern, die mindestens ein spezifisches Aminosilikon und ein Alkalisierungsmittel enthalten, die an diese Mittel gestellten Anforderungen in besonderem Ausmaß erfüllen.

Ein erster Gegenstand der vorliegenden Erfindung ist daher ein Mittel zum Färben und/oder Aufhellen von Haaren, welches dadurch gekennzeichnet ist, dass es in einem kosmetisch geeigneten Träger unter anderem

a. mindestens ein Alkalisierungsmittel und
b. mindestens eine Verbindung der Formel (I)

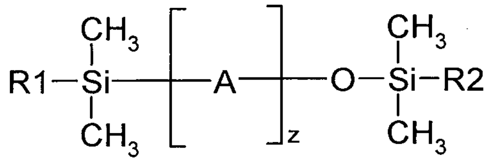

(I)

mit

A =

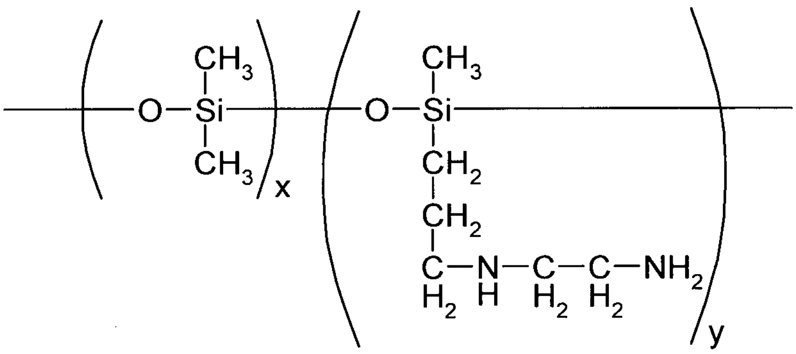

worin

i. x und y unabhängig von einander für Werte zwischen 1 und 100 stehen und
ii. z für Werte zwischen 1 und 100 steht, wobei, falls $z \geq 2$ ist, die jeweiligen Werte x und y in einem Strukturelement A jeweils unabhängig von vorangehenden Strukturelementen A gewählt werden können
und
R1 für $H_3C\text{-}(CH_2)_{15}\text{-}$ oder $H_3C\text{-}(CH_2)_{17}\text{-}$ und R2 für $H_3C\text{-}(CH_2)_{15}\text{-}$ oder $H_3C\text{-}(CH_2)_{17}\text{-}$ steht,

c. mindestens ein Oxidationsfarbstoffvorprodukt vom Entwickler- und/oder Kupplertyp enthält.

Ein weiterer Gegenstand der vorliegenden Erfindung ist ein Mittel zum Färben und/oder Aufhellen von Haaren, welches dadurch gekennzeichnet ist, dass es in einem kosmetisch geeigneten Träger

a. mindestens ein Oxidationsfarbstoffvorprodukt,
b. mindestens ein Alkalisierungsmittel gemäß Anspruch 2 und
c. mindestens eine Verbindung der Formel (I)

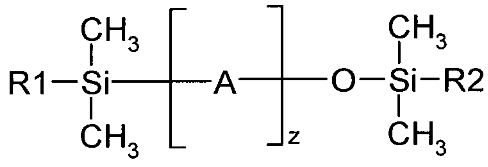

(I)

mit

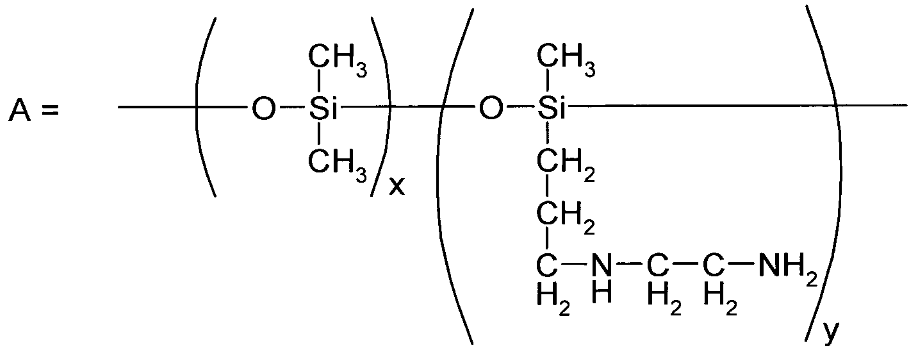

worin

i. x und y unabhängig von einander für Werte zwischen 1 und 100 stehen und
ii. z für Werte zwischen 1 und 100 steht, wobei, falls $z \geq 2$ ist, die jeweiligen Werte x und y in einem Strukturelement A jeweils unabhängig von vorangehenden Strukturelementen A gewählt werden können und
iii. R1 und R2 unabhängig voneinander für eine lineare oder verzweigte, gesättigte, ungesättigte oder mehrfach ungesättigte $C_5$-$C_{20}$-Alkylkette stehen,

enthält,
mit der Maßgabe, dass das Mittel nicht gleichzeitig Ammoniak und Kaliumhydroxid enthält.

Die erfindungsgemäßen Mittel enthalten die Verbindungen der Formel (I) in einem kosmetischen Träger, bevorzugt in einem geeigneten wässrigen, alkoholischen oder wässrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole, Schaum-formulierungen oder andere Zubereitungen, die für die Anwendung auf dem Haar geeignet sind. Es ist aber auch denkbar, die Farbstoffvorprodukte gemäß Formel (I) in eine pulverförmige oder auch tablettenförmige Formulierung zu integrieren.

Unter wässrig-alkoholischen Lösungen sind im Sinne der vorliegenden Erfindung wässrige Lösungen enthaltend 3 bis 70 Gew.-% eines $C_1$-$C_4$-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.
Die Strukturelemente A der Verbindung der Formel (I) sind erfindungsgemäß aus je einem oder mehreren Elementen 3-[(2-Aminoethyl)amino]propyl-methyl-siloxan und einem oder mehreren Elementen Dimethylsiloxan aufgebaut. Die Anzahl an Dimethylsiloxan Elementen wird durch den Parameter x definiert. Die Anzahl an 3-[(2-Aminoethyl)amino]propyl-methyl-siloxan Elementen wird durch den Parameter y definiert. Die Werte der Parameter x und y stehen erfindungsgemäß unabhängig voneinander für Zahlen zwischen 1 und 100.
Die Anzahl an Strukturelementen A wird durch den Parameter z vorgegeben. Erfindungsgemäß liegt der Wert des Parameters z zwischen 1 und 100. Falls $z \geq 2$, können die Parameter x und y in jedem Strukturelement A unabhängig von vorangehenden Strukturelementen A gewählt werden. Daraus folgt, dass sich für den Fall $z \geq 2$ die einzelnen Strukturelemente A in ihrer Anzahl an 3-[(2-Aminoethyl)amino]propyl-methyl-siloxan Elementen und/oder in ihrer Anzahl an Dimethylsiloxan Elementen voneinander unterscheiden können.

Ein erfindungsgemäßes Mittel ist dadurch gekennzeichnet, dass es eine Verbindung der Formel (I) enthält, bei welcher R1 für $H_3C$-$(CH_2)_{15}$- oder $H_3C$-$(CH_2)_{17}$- und R2 für $H_3C$-$(CH_2)_{15}$- oder $H_3C$-$(CH_2)_{17}$- steht. Solche Verbindungen sind unter der INCI-Bezeichnung Bis-Cetearyl Amodimethicon bekannt.

In einer weiteren Ausführungsform des ersten Erfindungsgegenstands ist ein erfindungsgemäßes Mittel dadurch gekennzeichnet, dass es, bezogen auf das Gesamtgewicht des Mittels, die Verbindung/en der Formel (I) in einem Gewichtsanteil von 0,005 bis 5 Gew.-%, bevorzugt 0,1 bis 4,5 Gew.-%, besonders bevorzugt 1 bis 3 Gew.-% und insbesondere bevorzugt 1,5 bis 2,5 Gew.-%, enthält.

Als weiteren wesentlichen Inhaltsstoff enthalten die erfindungsgemäßen Mittel ein Alkalisierungsmittel. Aufhell- bzw. Blondiermittel benötigen in der Regel einen basischen pH-Wert zur Ausfärbung, insbesondere zwischen pH 8,0 und pH 12. Diese pH-Werte sind notwendig, um eine Öffnung der äußeren Schuppenschicht (Cuticula) zu gewährleisten und eine Penetration der aktiven Spezies, insbesondere Wasserstoffperoxid, in das Haar zu ermöglichen.

Der benötigte basische pH-Wert wird häufig mit Ammoniak als Alkalisierungsmittel eingestellt, da ammoniakhaltige Färbe- und Blondiermittel zusätzliche Vorteile hinsichtlich der Grauabdeckung und der Aufhellleistung besitzen.

Für den Anwender besitzt ein solches Aufhellmittel jedoch den Nachteil, dass es durch Ammoniak neben zusätzlichen Schädigungen des Haares zu Reizungen von Augen oder Kopfhaut kommen kann, wodurch Sensibilisierungen oder gar allergische Reaktionen hervorgerufen werden können. Darüber hinaus besitzen solche Haarbehandlungsmittel einen intensiven, unangenehmen Geruch, der schlimmstenfalls auch zu Reizungen der Nasenschleimhaut führen kann. Außerdem kann auch die Produktion und Lagerung von Ammoniakhaltigen Mitteln mit Problemen hinsichtlich Handhabbarkeit und Stabilität verbunden sein. In einer bevorzugten Ausführungsform ist das erfindungsgemäße Mittel daher frei von Ammoniak.

In einer bevorzugten Ausführungsform des ersten Erfindungsgegenstands ist ein erfindungsgemäßes Mittel daher dadurch gekennzeichnet, dass es weniger als 1 Gew.-%, vorzugsweise weniger als 0,1 Gew.-%, besonders bevorzugt weniger als 0,01 Gew.-% und insbesondere bevorzugt weniger als 0,001 Gew.-% Ammoniak, bezogen auf das Gesamtgewicht des Mittels, enthält.

Die erfindungsgemäßen Mittel zum Behandeln von Haaren enthalten als Alkalisierungsmittel ein Alkanolamin, eine basische Aminosäure oder deren Gemische.

Üblicherweise wird das Alkalisierungsmittel bzw. das Alkalisierungsmittelgemisch in einer Menge zugesetzt, die die Einstellung des für die jeweilige Haarbehandlung optimalen pH-Wertes gewährleistet. Bevorzugt weisen die erfindungsgemäßen Mittel einen pH-Wert im Bereich von 4 bis 12 auf. Im Fall von Oxidationsfärbemitteln und Aufhellmitteln findet die Anwendung der Färbemittel vorzugsweise in einem alkalischen Milieu statt, bevorzugt bei einem pH-Wert im Bereich von 8,0 bis 12,0 und besonders bevorzugt von 9,0 bis 11,0. Bei den pH-Werten im Sinne der vorliegenden Erfindung handelt es sich um pH-Werte, die bei einer Temperatur von 22°C gemessen wurden.

Je nach Wahl des gewünschten pH-Wertes und abhängig vom Vorhandensein weiterer Komponenten im erfindungsgemäßen Mittel, wie beispielsweise sauerer oder basischer Salze oder Pufferkomponenten, kann die Menge es zugesetzten Alkalisierungsmittel variieren, üblicherweise sind hierfür Mengen von 0,01 bis 15 Gew.-% notwendig.

Das erfindungsgemäße Mittel ist daher dadurch gekennzeichnet, dass es, bezogen auf das Gesamtgewicht des Mittels, 0,01 bis 15 Gew.-%, vorzugsweise 0,1 bis 10 Gew.-%, besonders bevorzugt 1 bis 8 Gew.-% und insbesondere bevorzugt 3 bis 7 Gew.-% mindestens eines Alkalisierungsmittels aus der Gruppe der Alkanolamine, der basischen Aminosäuren oder deren Gemische enthält.

Die in dem erfindungsgemäßen Mittel einsetzbaren Alkanolamine werden bevorzugt ausgewählt aus primären Aminen mit einem $C_2$-$C_6$-Alkylgrundkörper, der mindestens eine Hydroxylgruppe trägt. Bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol.

Erfindungsgemäß besonders bevorzugte Alkanolamine werden ausgewählt aus 2-Aminoethan-1-ol und/oder 2-Amino-2-methylpropan-1-ol. Eine besonders bevorzugte Ausführungsform ist daher dadurch gekennzeichnet, dass das erfindungsgemäße Mittel als Alkalisierungsmittel ein Alkanolamin ausgewählt aus 2-Aminoethan-1-ol und/oder 2-Amino-2-methylpropan-1-ol enthält.

Als Aminosäure im Sinne der Erfindung gilt eine organische Verbindung, die in ihrer Struktur mindes-tens eine protonierbare Aminogruppe und mindestens eine -COOH- oder eine -$SO_3$H-Gruppe enthält. Bevorzugte Aminosäuren sind Aminocarbonsäuren, insbesondere $\alpha$-(alpha)-Aminocarbonsäuren und $\omega$-Aminocarbonsäuren, wobei $\alpha$-Aminocarbonsäuren besonders bevorzugt sind.

Unter basischen Aminosäuren sind erfindungsgemäß solche Aminosäuren zu verstehen, welche einen isoelektrischen Punkt pl von größer 7,0 besitzen.

Basische $\alpha$-Aminocarbonsäuren enthalten mindestens ein asymmetrisches Kohlenstoffatom. Im Rahmen der vorliegenden Erfindung können beide möglichen Enantiomere als spezifische Ver bindung oder auch deren Gemische, insbesondere als Racemate, gleichermaßen eingesetzt werden. Es ist jedoch besonders vorteilhaft, die natürlich bevorzugt vorkommende Isomerenform, üblicherweise in L-Konfiguration, einzusetzen.

Die basischen Aminosäuren werden bevorzugt ausgewählt aus der Gruppe, die gebildet wird aus Arginin, Lysin, Ornithin und Histidin, besonders bevorzugt aus Arginin und Lysin. In einer weiteren besonders bevorzugten Ausführungsform ist ein erfindungsgemäßes Mittel daher dadurch gekennzeichnet, dass es sich bei dem Alkalisierungsmittel um eine basische Aminosäure aus der Gruppe Arginin, Lysin, Ornithin und/oder Histidin handelt.

Bevorzugte erfindungsgemäße Mittel können ebenfalls Alkalisierungsmittelgemische wie beispielsweise ein Gemisch aus verschiedenen Alkanolaminen, ein Gemisch aus basischen Aminosäuren und/oder ein Gemisch aus Alkanolaminen und basischen Aminosäuren enthalten. Besonders bevorzugt werden die die Alkalisierungsmittel in bestimmten Kombinationen eingesetzt: 2-Aminoethan-1-ol / 2-Amino-2-methylpropan-1-ol; 2-Aminoethan-1-ol/Arginin; 2-Aminoethan-1-ol/Lysin; 2-Aminoethanl-1-ol/Ornithin; 2-Aminoethan-1-ol/Histidin; 2-Amino-2-methylpropan-1-ol / Arginin; 2-Amino-2-methylpropan-1-ol / Lysin; 2-Amino-2-methylpropan-1-ol / Ornithin; 2-Amino-2-methylpropan-1-ol / Histidin; Arginin / Lysin; Arginin / Ornithin; Arginin / Histidin; Lysin / Ornithin; Lysin / Histidin und/oder Orithin / Histidin.

Darüber hinaus kann das Mittel weitere Alkalisierungsmittel, insbesondere anorganische Alkalisierungsmittel

enthalten. Erfindungsgemäß einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Neben den zuvor beschriebenen Alkalisierunsmitteln sind dem Fachmann zur Einstellung des pH-Wertes gängige Acidifizierungsmittel geläufig. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren.

Die erfindungsgemäßen Mittel zum Färben und/oder Aufhellen von Haaren enthalten als weiteren Bestandteil neben der/den Verbindung(en) der Formel (I) und einem Alkalisierungsmittel mindestens ein Oxidationsfarbstoffvorprodukt vom Entwickler und/oder Kupplertyp.

Erfindungsgemäß sind solche Mittel bevorzugt, die die Entwickler und/oder Kupplerkomponenten jeweils in einem Gewichtsanteil von 0,001 bis 10 Gew.-%, bevorzugt, 0,01 bis 8 Gew.-%,
besonders bevorzugt 0,1 bis 5 Gew.-% und insbesondere bevorzugt 0,5 bis 3 Gew.-%, bezogen auf das Gesamtgewicht des Mittels, enthalten.

In einer weiteren bevorzugten Ausführungsform ist das erfindungsgemäße Mittel daher dadurch gekennzeichnet, dass es ein Oxidationsfarbstoffvorprodukt vom Entwickler und/oder Kupplertyp in einem Gewichtsanteil von 0,001 bis 10 Gew.-%, bevorzugt 0,01 bis 8 Gew.-%, besonders bevorzugt 0,1 bis 5 Gew.-% und insbesondere bevorzugt 0,5 bis 3 Gew.-%, bezogen auf das Gesamtgewicht des Mittels, enthält.

In einer bevorzugten Ausführungsform des ersten Erfindungsgegenstands enthält das Mittel als Oxidationsfarbstoffvorprodukt mindestens eine Entwicklerkomponente und gegebenenfalls mindestens eine Kupplerkomponente. Die Entwicklerkomponenten können untereinander, bevorzugt aber mit Kupplerkomponenten die eigentlichen Farbstoffe ausbilden. Vorzugsweise enthalten die erfindungsgemäßen Färbemittel daher mindestens ein Oxidationsfarbstoffvorprodukt vom Entwicklertyp und mindestens ein Oxidationsfarbstoffvorprodukt vom Kupplertyp. Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Hydrobromide oder der Sulfate einzusetzen.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 3 : 1 bis 1 : 3, insbesondere 2 : 1 bis 1 : 1, enthalten sein können.

Geeignete Oxidationsfarbstoffvorprodukte vom Entwicklertyp sind p-Phenylendiamin und dessen Derivate. Bevorzugte p-Phenylendiamine werden ausgewählt aus einer oder mehrerer Verbindungen der Gruppe, die gebildet wird aus p-Phenylendiamin, p-Toluylendiamin, 2-Chlor-p-phenylendiamin, 2,3-Dimethyl-p-phenylendiamin, 2,6-Dimethyl-p-phenylendiamin, N,N-Bis-(2-hydroxyethyl)-p-phenylen-diamin, 2-(2-Hydroxyethyl)-p-phenylendiamin, 2-(1,2-Dihydroxyethyl)-p-phenylendiamin, N-(2-Hydroxypropyl)-p-phenylendiamin, N-(4'-Aminophenyl)-p-phenylendiamin, 2-Methoxymethyl-p-phenylendiamin, N-Phenyl-p-phenylendiamin, 2-(2-Hydroxyethyloxy)-p-phenylendiamin und N-(4-Amino-3-methyl-phenyl)-N-[3-(1H-imidazol-1-yl)propyl]amin sowie ihren physiologisch verträglichen Salzen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind. Bevorzugte zweikernige Entwicklerkomponenten werden ausgewählt N,N'-Bis-(2-hydroxyethyl)-N,N'-bis-(4'-aminophenyl)-1,3-diaminopropan-2-ol, N,N'-Bis-(4-aminophenyl)-1,4-diazacycloheptan und Bis-(2-hydroxy-5-aminophenyl)methan sowie deren physiologisch verträglichen Salzen.

Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind p-Aminophenol, N-Methyl-p-Aminophenol, 4-Amino-3-methylphenol, 4-Amino-2-aminomethyl-phenol, 4-Amino-2-(1,2-dihydroxyethyl)phenol und 4-Amino-2-(diethylamino-methyl)phenol sowie deren physiologisch verträglichen Salze.

Ferner kann die Entwicklerkomponente ausgewählt sein aus o-Aminophenol und seinen Derivaten, bevorzugt aus 2-Amino-4-methylphenol, 2-Amino-5-methylphenol, 2-Amino-4-chlorphenol und/oder deren physiologisch verträglichen Salzen.

Weiterhin kann die Entwicklerkomponente ausgewählt sein aus heterocyclischen Entwicklerkomponenten, wie Pyrimidin-Derivaten, Pyrazol-Derivaten, Pyrazolopyrimidin-Derivaten bzw. ihren physiologisch verträglichen Salzen. Bevorzugte Pyrimidin-Derivate sind die Verbindungen 2,4,5,6-Tetraaminopyrimidin, 4-Hydroxy-2,5,6-triaminopyrimidin und deren physiologisch verträglichen Salze. Bevorzugtes Pyrazol-Derivat ist 4,5-Diamino-1-(2-hydroxyethyl)pyrazol sowie dessen physiologisch verträglichen Salze. Als Pyrazolopyrimidine sind insbesondere Pyrazolo[1,5-a]pyrimidine bevorzugt.

Bevorzugte Oxidationsfarbstoffvorprodukte vom Entwicklertyp sind ausgewählt aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxy-ethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-amino-methylphenol, 4-Amino-2-(1,2-dihydroxyethyl)phenol und 4-Amino-2-(diethylaminomethyl)phenol, 4,5-Diamino-1-(2-hydroxyethyl)-pyrazol, 2,4,5,6-Tetraaminopyrimidin, 4-Hydroxy-2,5,6-triaminopyrimidin, 2-Hydroxy-4,5,6-triaminopyrimidin oder den physiologisch verträglichen Salzen dieser Verbindungen.

Besonders bevorzugte Entwicklerkomponenten sind p-Toluylendiamin, 2-(2-Hydroxyethyl)-p-phenylen-diamin, 2-Methoxymethyl-p-phenylendiamin, N-(4-Amino-3-methylphenyl)-N-[3-(1H-imidazol-1-yl)-propyl]amin, und/oder 4,5-Diamino-1-(2-hydroxyethyl)pyrazol sowie deren physiologisch verträglichen Salze.

Eine weitere bevorzugte Ausführungsform der vorliegenden Erfindung ist dadurch gekennzeichnet, dass das erfindungsgemäße Mittel als Oxidationsfarbstoffvorprodukt neben mindestens einer Entwicklerkomponente weiterhin zusätzlich mindestens eine Kupplerkomponente enthält. Als Kupplerkomponenten werden in der Regel m-Phenylendiaminderivate, Naphthole, Resorcin und Resorcinderivate, Pyrazolone und m-Aminophenolderivate verwendet.

Erfindungsgemäß bevorzugte Kupplerkomponenten sind

(A) m-Aminophenol und dessen Derivate, insbesondere 3-Aminophenol, 5-Amino-2-methylphenol, 3-Amino-2-chlor-6-methylphenol, 5-Amino-4-chlor-2-methylphenol, 5-(2'-Hydroxyethyl)-amino-2-methylphenol und 2,4-Dichlor-3-aminophenol,

(B) o-Aminophenol und dessen Derivate, wie 2-Amino-5-ethylphenol,

(C) m-Diaminobenzol und dessen Derivate wie beispielsweise 2,4-Diaminophenoxy-ethanol, 1,3-Bis-(2',4'-diaminophenoxy)-propan, 1-Methoxy-2-amino-4-(2'-hydroxyethylamino)benzol, 2,6-Bis-(2'-hydroxyethylamino)-1-methylbenzol, 2-({3-[(2-Hydroxyethyl)amino]-4-methoxy-5-methylphenyl}amino)ethanol und 2-({3-[(2-Hydroxyethyl)amino]-2-methoxy-5-methylphenyl}-amino)ethanol,

(D) o-Diaminobenzol und dessen Derivate,

(E) Di- beziehungsweise Trihydroxybenzolderivate, insbesondere Resorcin, 2-Chlorresorcin, 4-Chlorresorcin, 2-Methylresorcin und 1,2,4-Trihydroxybenzol,

(F) Pyridinderivate, insbesondere 3-Amino-2-methylamino-6-methoxypyridin, 2,6-Diaminopyridin, 2,6-Dihydroxy-3,4-dimethylpyridin, 2-Amino-3-hydroxypyridin und 3,5-Diamino-2,6-dimethoxy-pyridin,

(G) Naphthalinderivate, wie 1-Naphthol und 2-Methyl-1-naphthol,

(H) Morpholinderivate, wie 6-Hydroxybenzomorpholin,

(I) Chinoxalinderivate,

(J) Pyrazolderivate, wie 1-Phenyl-3-methylpyrazol-5-on,

(K) Indolderivate, wie 6-Hydroxyindol,

(L) Pyrimidinderivate oder

(M) Methylendioxybenzolderivate, wie 1-(2'-Hydroxyethyl)-amino-3,4-methylendioxybenzol sowie deren physiologisch verträglichen Salze.

Erfindungsgemäß bevorzugte Kupplerkomponenten sind ausgewählt aus der Gruppe, die gebildet wird aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis-(2,4-diaminophenyl)propan, 2,6-Bis(2'-hydroxyethylamino)-1-methylbenzol, 2-({3-[(2-Hydroxyethyl)-amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder den physiologisch verträglichen Salzen der vorgenannten Verbindungen.

Erfindungsgemäß besonders bevorzugte Kupplerkomponenten sind Resorcin, 2-Methylresorcin, 5-Amino-2-methylphenol, 3-Aminophenol, 2-(2,4-Diaminophenoxy)ethanol, 1,3-Bis-(2,4-diaminophenoxy)propan, 1-Methoxy-2-amino-4-(2'-hydroxyethylamino)benzol, 2-Amino-3-hydroxypyridin, 2,6-Dihydroxy-3,4-dimethylpyridin, 1,5-Dihydroxynaphthalin, 2,7-Dihydroxynaphthalin und 1-Naphthol sowie eines deren physiologisch verträglichen Salze.

In einer weiteren Ausführungsform des ersten Erfindungsgegenstands ist ein erfindungsgemäßes Mittel dadurch gekennzeichnet, dass es als Oxidationsfarbstoffvorprodukt mindestens eine Entwickler-komponenten aus der Gruppe enthält, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxy-ethyl)phenol und 4-Amino-2-(diethylaminomethyl)-phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin oder den physiologisch verträglichen Salzen dieser Verbindungen, sowie gegebenenfalls zusätzlich mindestens eine Kupplerkomponente aus der Gruppe enthält, die gebildet wird aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)-propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}-amino)ethanol, 2-({3-[(2-Hydroxyethyl)amino]-2-methoxy-5-methylphenyl} amino)ethanol, 2-({3-[(2-Hydroxyethyl)amino]-4,5-dimethylphenyl}amino)ethanol, 2-[3-Morpholin-4-ylphenyl)amino]-ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder den physiologisch verträglichen Salzen der vorgenannten Verbindungen.

Oxidationsfarbstoffvorprodukte vom Entwicklertyp und vom Kupplertyp werden besonders bevorzugt in bestimmten Kombinationen eingesetzt. Mit den als Kombination genannten Oxidationsfarbstoffvorprodukten und/oder deren physiologisch verträglichen Salzen können jedoch auch noch weitere Farbstoffvorprodukte kombiniert werden: p-Toluylendiamin / Resorcin; p-Toluylendiamin / 2-Methyl-resorcin; p-Toluylendiamin / 5-Amino-2-methylphenol; p-Toluylendiamin / 3-Aminophenol; p-Toluylen-diamin / 2-(2,4-Diaminophenoxy)ethanol; p-Toluylendiamin / 1,3-Bis(2,4-diaminophenoxy)propan; p-Toluylendiamin / 1-Methoxy-2-amino-4-(2-hydroxyethylamino)benzol; p-Toluylendiamin / 2-Amino-3-hydroxypyridin;p-Toluylendiamin / 1-Naphthol; 2-(2-Hydroxyethyl)-p-phenylendiamin / Resorcin; 2-(2-Hydroxyethyl)-p-phenylendiamin / 2-Methylresorcin; 2-(2-Hydroxyethyl)-p-phenylendiamin / 5-Amino-2-methylphenol; 2-(2-Hydroxyethyl)-p-phenylendiamin / 3-Aminophenol; 2-(2-Hydroxyethyl)-p-phenylendiamin / 2-(2,4-Diaminophenoxy)ethanol; 2-(2-Hydroxyethyl)-p-phenylendiamin / 1,3-Bis-(2,4-diaminophenoxy)propan; 2-(2-Hydroxyethyl)-p-phenylendiamin / 1-Methoxy-2-amino-4-(2-hydroxyethylamino)benzol; 2-(2-Hydroxyethyl)-p-phenylendiamin / 2-Amino-3-hydroxypyridin; 2-(2-Hydroxyethyl)-p-phenylendiamin / 1-Naphthol; 2-Methoxymethyl-p-phenylendiamin / Resorcin; 2-Methoxymethyl-p-phenylendiamin / 2-Methylresorcin; 2-Methoxymethyl-p-phenylendiamin / 5-Amino-2-methylphenol; 2-Methoxymethyl-p-phenylendiamin / 3-Aminophenol; 2-Methoxymethyl-p-phenylendiamin / 2-(2,4-Diaminophenoxy)ethanol; 2-Methoxymethyl-p-phenylendiamin / 1,3-Bis(2,4-diaminophenoxy)propan; 2-Methoxymethyl-p-phenylendiamin / 1-Methoxy-2-amino-4-(2-hydroxyethylamino)benzol; 2-Methoxymethyl-p-phenylendiamin / 2-Amino-3-hydroxypyridin; 2-Methoxymethyl-p-phenylendiamin / 1-Naphthol; N-(4-Amino-3-methylphenyl)-N-[3-(1H-imidazol-1-yl)propyl]amin / Resorcin; N-(4-Amino-3-methylphenyl)-N-[3-(1H-imidazol-1-yl)propyl]amin / 2-Methylresorcin; N-(4-Amino-3-methylphenyl)-N-[3-(1H-imidazol-1-yl)propyl]amin / 5-Amino-2-methylphenol; N-(4-Amino-3-methylphenyl)-N-[3-(1H-imidazol-1-yl)propyl]amin / 3-Aminophenol; N-(4-Amino-3-methylphenyl)-N-[3-(1H-imidazol-1-yl)propyl]amin / 2-(2,4-Diaminophenoxy)ethanol; N-(4-Amino-3-methylphenyl)-N-[3-(1H-imidazol-1-yl)propyl]amin / 1,3-Bis(2,4-diaminophenoxy)-propan; N-(4-Amino-3-methylphenyl)-N-[3-(1H-imidazol-1-yl)propyl]amin / 1-Methoxy-2-amino-4-(2-hydroxyethylamino)benzol; N-(4-Amino-3-methylphenyl)-N-[3-(1H-imidazol-1-yl)propyl]amin / 2-Amino-3-hydroxypyridin; N-(4-Amino-3-methylphenyl)-N-[3-(1H-imidazol-1-yl)propyl]amin / 1-Naphthol; 4,5-Diamino-1-(2-hydroxyethyl)pyrazol / Resorcin; 4,5-Diamino-1-(2-hydroxyethyl)pyrazol / 2-Methylresorcin; 4,5-Diamino-1-(2-hydroxyethyl)pyrazol / 5-Amino-2-methylphenol; 4,5-Diamino-1-(2-hydroxyethyl)pyrazol / 3-Aminophenol; 4,5-Diamino-1-(2-hydroxyethyl)pyrazol / 2-(2,4-Diaminophenoxy)ethanol; 4,5-Diamino-1-(2-hydroxyethyl)pyrazol / 1,3-Bis(2,4-diaminophenoxy)-propan; 4,5-Diamino-1-(2-hydroxyethyl)pyrazol / 1-Methoxy-2-amino-4-(2-hydroxyethylamino)-benzol; 4,5-Diamino-1-(2-hydroxyethyl)pyrazol / 2-Amino-3-hydroxypyridin; 4,5-Diamino-1-(2-hydroxyethyl)pyrazol / 1-Naphthol.

In einer weiteren Ausführungsform der vorliegenden Erfindung enthalten die erfindungsgemäßen Mittel weiterhin zusätzlich mindestens eine Vorstufe eines naturanalogen Farbstoffs. Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Besonders bevorzugte Derivate des Indolins sind das 5,6-Dihydroxyindolin und das 2,3-Dioxoindolin (Isatin) und deren physiologisch verträglichen Salze. Ein besonders bevorzugtes Derivat des Indols ist das 5,6-Dihydroxyindol und dessen physiologisch verträglichen Salze. Die erfindungsgemäßen Mittel enthalten die Indol- oder Indolin-Derivate vorzugsweise in einem Gewichtsanteil von 0,05 bis 10 Gew.-%, bevorzugt 0,2 bis 5 Gew.-%, jeweils bezogen auf ihr Gesamtgewicht.

Im Falle der oxidativen Färbungen kann die Entwicklung der Farbe grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist.

Ferner können die erfindungsgemäßen Mittel weitere Wirk-, Hilfs- und Zusatzstoffe enthalten, wie beispielsweise Assoziativpolymere mit Fettalkylkette, kationische Polymere, nichtionische Polymere (Vinylpyrrolidinon/Vinylacrylat-Copolymere, Polyvinylpyrrolidinon, Vinylpyrrolidinon/Vinylacetat-Copolymere, Polyethylenglykole und Polysiloxane); zwitterionische und amphotere Polymere (Acrylamidopropyl-trimethylammoniumchlorid/Acrylat-Copolymere und Octylacrylamid/-Methyl-methacrylat/tert-Butylaminoethylmethacrylat/2-Hydroxypropylmethacrylat-Copolymere); anionische Polymere (Polyacrylsäuren, vernetzte Polyacrylsäuren, Vinylacetat/Crotonsäure-Copolymere, Vinylpyrrolidinon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert-Butyl-acrylamid-Terpolymere); Verdickungsmittel (Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol); Strukturanden (Glusose, Maleinsäure und Milchsäure), haarkon-ditionierende Verbindungen (Phospholipide, Sojalecithin, Ei-Lecitin, Kephaline sowie Silikonöle); zusätzliche Proteinhydrolysate pflanzlicher oder tierischer Herkunft (Elastin-, Collagen-, Keratin-, Milcheiweiß-, Sojaprotein- und Weizenproteinhydrolysate, deren Kondensationsprodukte mit Fettsäuren sowie quaternisierte Proteinhydrolysate); Parfümöle, Dimethylisosorbid und Cyclodextrine; faserstrukturverbessernde Wirkstoffe (Mono-, Di- und Oligosaccharide, Glucose, Maleinsäure und Milchsäure); Entschäumer wie Silikone (Dimethicon); Farbstoffe zum Anfärben des Mittels; Antischuppenwirkstoffe (Piroctone Olamine, Zink Omadine und Climbazol); Lichtschutzmittel (derivatisierte Benzophenone, Zimtsäure-Derivate und Triazine); Wirkstoffe (Panthenol, Panthothensäure, Pantolacton, Allantoin, Pyrrolidinoncarbonsäuren und deren Salze, Bisabolol, Carnitin, Coffein, Theobromin und Taurin); Vitamine, Provitamine und Vitaminvorstufen, insbesondere solche der Gruppen A, $B_3$, $B_5$, $B_6$, C, E, F und H; Pflanzenextrakte (aus Grünem Tee, Eichenrinde, Brennnessel, Hamamelis, Hopfen, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Litchi, Weizen, Kiwi, Melone, Orange, Grapefruit, Salbei, Rosmarin, Birke, Malve, Wiesenschaumkraut, Quendel, Schafgarbe, Thymian, Melisse, Moringa, Hauhechel, Huflattich, Eibisch, Meristem, Ginseng und Ingwer); pflanzliche Öle (Macadamianussöl, Kukuinussöl, Palmöl, Amaranthsamenöl, Pfirsichkernöl, Avocadoöl, Olivenöl, Kokosöl, Rapsöl, Sesamöl, Jojobaöl, Sojaöl, Erdnussöl, Nachtkerzenöl, Teebaumöl); Cholesterin; Konsistenzgeber (Zuckerester, Polyolester oder Polyolalkylether); Fette und Wachse (Fettalkohole, Bienenwachs, Montanwachs und Paraffine); Quell- und Penetrationsstoffe (Glycerin, Propylenglykolmonoethylether, Carbonate, Hydrogencarbonate, Guanidine, Harnstoffe sowie primäre, sekundäre und tertiäre Phosphate); Trübungsmittel (Latex, Styrol/PVP- und Styrol / Acrylamid-Copolymere); Perlglanzmittel (Ethylenglykolmonostearat sowie PEG-3-distearat); Treibmittel wie Propan-Butan-Gemische, $N_2O$, Dimethylether, $CO_2$ und Luft; Antioxidantien.

Bevorzugt ist es, wenn das erfindungsgemäße Mittel zusätzlich mindestens einen Wirk-, Hilfs- und Zusatzstoff aus der Gruppe enthält, die gebildet wird aus

a. Strukturanten ausgewählt aus Glucose, Maleinsäure und Milchsäure,
b. haarkonditionierenden Verbindungen ausgewählt aus Phospholipiden, Sojalecithin, Ei-Lecitin, Kephalinen und Silikonölen,
c. Proteinhydrolysaten ausgewählt aus Elastin-, Kollagen-, Keratin-, Milcheiweiß-, Sojaprotein- und Weizenproteinhydrolysaten, deren Kondensationsprodukte mit Fettsäuren sowie quaternisierten Proteinhydrolysaten,
d. Wirkstoffen ausgewählt aus Panthenol, Pantothensäure, Pantolacton, Allantoin, Pyrrolidoncarbonsäuren und deren Salzen, Bisabolol, Carnitin, Coffein, Theophyllin, Theobromin und Taurin,
e. Vitaminen, Provitaminen und Vitaminvorstufen und
f. Pflanzenextrakten.

Besonders vorteilhafte Effekte lassen sich erzielen, wenn das erfindungsgemäße Mittel mindestens einen zusätzlichen Wirkstoffe aus der Gruppe enthält, die gebildet wird aus Panthenol, Pantothensäure, Pantolacton, Allantoin, Bisabolol, Carnitin, Coffein, Theophyllin, Theobromin und Taurin.

Innerhalb dieser Gruppe wiederum ganz besonders bevorzugt ist es, wenn das erfindungsgemäße Mittel zusätzlich mindestens einen Wirkstoff ausgewählt aus Coffein, Theophyllin, Theobromin und/oder Taurin, enthält.

In einer weiteren bevorzugten Ausführungsform ist das erfindungsgemäße Mittel daher dadurch gekennzeichnet, dass es zusätzlich einen Wirkstoff ausgewählt aus Panthenol, Pantothensäure, Pantolacton, Allantoin, Bisabolol, Carnitin, Coffein, Theophyllin, Theobromin und/oder Taurin, bevorzugt ausgewählt aus Coffein, Theophyllin, Theobromin und/oder Taurin, enthält.

Die Auswahl dieser weiteren Stoffe wird der Fachmann gemäß den gewünschten Eigenschaften der Zubereitungen treffen. Die Zubereitungen enthalten die weiteren Wirk-, Hilfs- und Zusatzstoffe bevorzugt in einem Gewichtsanteil von 0,01 bis 25 Gew.-%, insbesondere 0,05 bis 15 Gew.-%, bezogen auf die Gesamtmenge des anwendungsbereiten Mittels.

Um eine vorzeitige, unerwünschte Reaktion der Oxidationsfarbstoffvorprodukte durch das Oxidationsmittel zu verhindern, werden Oxidationsfarbstoffvorprodukte und Oxidationsmittel selbst zweckmäßigerweise getrennt voneinander konfektioniert und erst unmittelbar vor der Anwendung in Kontakt gebracht.

In einer weiteren Ausführungsform der vorliegenden Erfindung sind daher Mittel bevorzugt, welche dadurch gekennzeichnet sind, dass sie unmittelbar vor der Anwendung durch Vermischen mindestens zweier Zubereitungen hergestellt werden, wobei die mindestens zwei Zubereitungen in mindestens zwei getrennt konfektionierten Containern bereitgestellt werden und wobei ein Container ein Färbe- und/oder Aufhellmittel (A) gemäß des ersten Erfindungsgegenstandes enthält, und ein weiterer Container eine Oxidationsmittelzubereitung (B), enthaltend mindestens ein Oxidationsmittel, enthält.

Als Oxidationsmittel können Wasserstoffperoxid sowie seine festen Anlagerungsprodukte an organische und anorganische Verbindungen eingesetzt werden. Als feste Anlagerungsprodukte kommen erfindungsgemäß insbesondere die Anlagerungsprodukte an Harnstoff, Melamin, Polyvinylpyrrolidinon sowie Natriumborat in Frage. Wasserstoffperoxid und/oder eines seiner festen Anlagerungsprodukte an organische oder anorganische Verbindungen sind als Oxidationsmittel besonders bevorzugt.

Insbesondere bevorzugt wird als Oxidationsmittel Wasserstoffperoxid eingesetzt. Bevorzugt beträgt der Gewichtsanteil an Wasserstoffperoxid im anwendungsbereiten Mittel 0,5 bis 12 Gew.-%, vorzugsweise 2 bis 10 Gew.-% und insbesondere bevorzugt 3 bis 6 Gew.-% (berechnet als 100 %-iges $H_2O_2$), jeweils bezogen auf das Gesamtgewicht des Mittels.

Ein weitere bevorzugte Ausführungsform des ersten Erfindungsgegenstands ist daher ein anwendungsbereites Mittel, welches dadurch gekennzeichnet ist, dass es zusätzlich als Oxidationsmittel Wasserstoffperoxid und/oder eines seiner festen Anlagerungsprodukte an organische oder anorganische Verbindungen in einem Gewichtsanteil von 0,5 bis 12 Gew.-%, vorzugsweise 2 bis 10 Gew.-% und insbesondere bevorzugt 3 bis 6 Gew.-% (berechnet als 100 %-iges $H_2O_2$), bezogen auf das Gesamtgewicht des Mittels, enthält.

Das anwendungsbereite Mittel kann in einer besonderen Ausführungsform der Erfindung erst kurz vor der Anwendung aus einer Farbveränderungszubereitung und einer Oxidationsmittelzubereitung hergestellt werden.

Solche Oxidationsmittelzubereitungen sind vorzugsweise wässrige Oxidationsmittelzubereitungen. Dabei sind bevorzugte Zubereitungen dadurch gekennzeichnet, dass die Oxidationsmittelzubereitung, bezogen auf ihr Gewicht, 40 bis 95 Gew.-%, vorzugsweise 50 bis 90 Gew.-%, besonders bevorzugt 55 bis 80 Gew.-% Wasser enthält.

Erfindungsgemäß kann aber das Farbveränderungsmittel als Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z. B. bestimmte Enzyme, Iodide, Chinone oder Metallionen.

Weiterhin hat es sich als vorteilhaft erweisen, wenn die Mittel, insbesondere die Oxidationsmittelzubereitungen, mindestens einen Stabilisator oder Komplexbildner enthalten. Im Rahmen der vorliegenden Erfindung bevorzugte Chelatkomplexbildner sind beispielsweise Polycarbonsäuren, stickstoffhaltige Mono- oder Polycarbonsäuren, insbesondere Ethylendiamintetraessigsäure (EDTA), Ethylendiamindibernsteinsäure (EDDS) und Nitrilotriessigsäure (NTA), geminale Diphosphonsäuren, insbesondere 1-Hydroxyethan-1,1-diphosphonsäure (HEDP), Aminophosphonsäuren wie Ethylendiamintetra(methylenphosphonsäure) (EDTMP) und Diethylentriaminpenta-(methylenphosphonsäure) (DTPMP), Phosphonopolycarbonsäuren wie 2-Phosphonobutan-1,2,4-tricarbonsäure sowie Cyclodextrine, Alkalistannate (Natriumstannat), Alkalipyrophosphate (Tetranatriumpyrophosphat, Dinatriumpyrophosphat), Alkaliphosphate (Natriumphosphat), und Phosphorsäure. Erfindungsgemäß bevorzugt enthalten die Mittel Komplexbildner zu 0,01 bis 3 Gew.-%, bevorzugt 0,05 bis 1 Gew.-%, jeweils bezogen auf das Gesamtgewicht des erfindungsgemäßen Mittels.

Für die starke Aufhellung sehr dunklen Haares ist der alleinige Einsatz von Wasserstoffperoxid oder dessen Anlagerungsprodukten an organische beziehungsweise anorganische Verbindungen oftmals nicht ausreichend. In diesen Fällen wird in der Regel eine Kombination aus Wasserstoffperoxid und Aufhellmitteln eingesetzt, woraus eine Steigerung des Aufhellvermögens der Mittel resultiert. Bevorzugte Aufhellmittel sind Bleichkraftverstäker. Hierzu zählen Peroxo-Salze, Siliciumdioxid-Verbindungen sowie insbesondere kationisierte Heterocyclen.

Vorzugsweise ist der Bleichkraftverstärker ausgewählt aus Ammoniumpersulfat, Alkalimetallpersulfaten, Ammoniumperoxomonosulfat, Alkalimetallhydrogenperoxomonosulfaten, Alkalimetallperoxodiphosphaten und Erdalkalimetallperoxiden. Besonders bevorzugte Bleichkraftverstärker sind Ammoniumperoxodisulfat, Kaliumperoxodisulfat, Natriumperoxodisulfat, Kaliumhydrogenperoxomonosulfat, Kaliumperoxodiphosphat, Magnesiumperoxid und Bariumperoxid. Erfindungsgemäß besonders bevorzugt sind Mittel, die als Bleichkraftverstärker mindestens ein anorganisches Salz, ausgewählt aus Peroxodisulfaten, enthalten. Weiterhin hat es sich bei den Arbeiten zur vorliegenden Erfindung als besonders bevorzugt erwiesen, wenn die erfindungsgemäßen Mittel mindestens zwei verschiedene Peroxodisulfate enthalten. Bevorzugte Peroxodisulfatsalze sind dabei Kombinationen aus Ammoniumperoxodisulfat und Kaliumperoxodisulfat und/oder Natriumperoxodisulfat.

Der Einsatz von Persulfatsalzen bzw. Peroxodisulfatsalzen erfolgt in der Regel in Form eines gegebenenfalls entstaubten Pulvers.

Die erfindungsgemäßen Zusammensetzungen können anstelle und/oder zusätzlich zu den festen Peroxo-Salzen einen weiteren Bleichkraftverstärker enthalten. Als Bleichkraftverstärker können Verbindungen, die unter Perhydrolysebedingungen aliphatische Peroxocarbonsäuren mit vorzugsweise 1 bis 10 C-Atomen, insbesondere 2 bis 4

C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. i-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Zur weiteren Steigerung der Leistung der Oxidationsmittelzubereitung kann der erfindungsgemäßen Zusammensetzung daher zusätzlich mindestens eine gegebenenfalls hydratisierte $SiO_2$-Verbindung zugesetzt werden. Es kann erfindungsgemäß bevorzugt sein, die gegebenenfalls hydratisierten $SiO_2$-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Hinsichtlich der gegebenenfalls hydratisierten $SiO_2$-Verbindungen unterliegt die vorliegende Erfindung prinzipiell keinen Beschränkungen. Bevorzugt sind Kieselsäuren, deren Oligomeren und Polymeren sowie deren Salze. Die gegebenenfalls hydratisierten $SiO_2$-Verbindungen können in verschiedenen Formen vorliegen. Erfindungsgemäß bevorzugt werden die $SiO_2$-Verbindungen in Form von Kieselgelen (Silicagel) oder als Wasserglas eingesetzt. Erfindungsgemäß besonders bevorzugt sind Wassergläser. Erfindungsgemäß besonders bevorzugte Wassergläser werden unter den Bezeichnungen Ferrosil 119, Natronwasserglas 40/42, Portil A, Portil AW, Portil W und Britesil C20 vertrieben.

Geeignete Bleichkraftverstärker vom Typ kationisierter Heterocyclen sind insbesondere physiologisch verträgliche Salze von Acetyl-1-methylpyridinium, 4-Acetyl-1-methylpyridinium und N-Methyl-3,4-dihydroisochinolinium, besonders bevorzugt 2-Acetyl-1-methylpyridinium-p-toluolsulfonat, 4-Acetyl-1-methylpyridinium-p-toluolsulfonat und N-Methyl-3,4-dihydroisochinolinium-p-toluolsulfonat.

Die Aufhellmittel sind in dem Mittel bevorzugt in einem Gewichtsanteil von 0,1 bis 25 Gew.-%, insbesondere in einer Menge von 0,5 bis 15 Gew.-%, bezogen auf das Gesamtgewicht des anwendungsbereiten Mittels, enthalten.

Bezüglich weiterer fakultativer Wirk- und Inhaltsstoffe der Oxidationsmittelzubereitung gilt mutatis mutandis das zu den erfindungsgemäßen Mitteln Gesagte.

Die erfindungsgemäß verwendbaren Mittel werden bevorzugt als fließfähige Zubereitungen formuliert. Dazu gehören insbesondere Emulsionen, Suspensionen und Gele, besonders bevorzugt Emulsionen. Bevorzugt enthalten die fließfähigen Zubereitungen zusätzlich als oberflächenaktive Substanz ein Emulgator bzw. ein Tensid, wobei oberflächenaktive Substanzen je nach Anwendungsgebiet als Tenside oder als Emulgatoren bezeichnet werden und aus anionischen, kationischen, amphoteren, zwitterionischen und nichtionischen Tensiden ausgewählt sind.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit etwa 8 bis 30 C-Atomen, bevorzugt 8 bis 24 C-Atomen. Zusätzlich können im Molekül Glykol- oder Polyglykolether-Gruppen, Ester-, Ether- und Amidgruppen sowie Hydroxylgruppen enthalten sein.

Bevorzugte anionische Tenside sind Seifen, Alkylsulfate, Alkylethersulfate und Ethercarbonsäuren mit 8 bis 22 C-Atomen in der Alkylgruppe und bis zu 12 Glykolethergruppen im Molekül. Als zwitterionische Tenside werden solche oberflächenaktiven Verbindungen bezeichnet, die im Molekül mindestens eine quartäre Ammoniumgruppe und mindestens eine Carboxylat-, Sulfonat- oder Sulfat-Gruppe tragen. Besonders geeignete zwitterionische Tenside sind die sogenannten Betaine wie die N-Alkyl-N,N-dimethylammonium-glycinate, N-Acyl-aminopropyl-N,N-dimethyl-ammoniumglycinate und 2-Alkyl-3-carboxymethyl-3-hydroxyethyl-imidazoline sowie Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat. In einer weiteren Ausführungsform der vorliegenden Erfindung enthält das Mittel weiterhin mindestens ein amphoteres Tensid. Unter amphoteren Tensiden werden solche oberflächenaktiven Verbindungen verstanden, die außer einer $C_8$-$C_{24}$-Alkyl- oder -Acylgruppe im Molekül mindestens eine freie Aminogruppe und mindestens eine COOH- oder $SO_3H$-Gruppe enthalten und zur Ausbildung innerer Salze befähigt sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside werden unter der INCI-Bezeichnung Disodium Cocoamphodipropionate mit den Handelsnamen Miranol C2M SF conc. (Rhodia), Amphoterge K-2 (Lonza) und Monateric CEM-38 (Unichema) und Bezeichnung Disodium Cocoamphodiacetate mit den Handelsnamen Dehyton (Cognis), Miranol C2M (Rhodia) und Ampholak XCO 30 (Akzo Nobel) vermarktet. Weiterhin hat es sich als vorteilhaft erwiesen, wenn die erfindungsgemäßen Färbe- oder Aufhellmittel nichtionogene grenzflächenaktive Stoffe enthalten. Nichtionische Tenside enthalten als hydrophile Gruppe z. B. eine Polyolgruppe, eine Polyalkylenglykolethergruppe oder eine Kombination aus Polyol- und Polyglykolethergruppe. Als nichtionische Tenside eignen bevorzugt sich Alkylpolyglykoside, insbesondere $C_8$-$C_{22}$-Alkylmono- und -oligoglycoside und deren ethoxylierte Analoga. Als weitere bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Erfindungsgemäß besonders bevorzugt sind gesättigte oder ungesättigte $C_{10}$-$C_{22}$ Fettalkohole mit jeweils 2 bis 12 Mol Ethylenoxid pro Mol Fettalkohol (z.B. Laureth-2 oder Ceteareth-20). Zubereitungen mit hervorragenden Eigenschaften werden ebenfalls erhalten, wenn sie als nichtionische Tenside Fettsäureester von ethoxyliertem Glycerin enthalten. Die anionischen, nichtionischen, amphoteren oder zwitterionischen Tenside werden in Gesamtmengen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugt sind ebenfalls kationische Tenside vom Typ der quartären Ammoniumverbindungen, der Esterquats und der Alkylamidoamine. Bevorzugte quaternäre Ammoniumverbindungen sind Ammoniumhalogenide, wie Alkyltrimethylammoniumchloride, Dialkyldimethylammoniumchloride und Trialkylmethylammoniumchloride, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Weitere erfindungsgemäß verwendbare kationische Tenside sind quaternisierte Proteinhydrolysate. Alkylamidoamine werden üblicherweise durch Amidierung natürlicher oder synthetischer Fettsäuren und Fettsäureschnitte mit Dialkylaminoaminen hergestellt. Eine erfindungsgemäß geeignete Verbindung aus dieser Substanzgruppe stellt Tegoamid® S 18 (Stearamidopropyldimethylamin) dar. Bei Esterquats handelt es sich um Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden unter den Warenzeichen Stepantex, Dehyquart und Armocare vertrieben. Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Die erfindungsgemäßen Mittel können auch direkt vor der Anwendung aus zwei oder mehreren getrennt verpackten Zubereitungen hergestellt werden. Dies bietet sich insbesondere zur Trennung inkompatibler Inhaltsstoffe an, um eine vorzeitige Reaktion zu vermeiden. Eine Auftrennung in Mehrkomponentensysteme bietet sich insbesondere dort an, wo Inkompatibilitäten der Inhaltsstoffe zu erwarten oder zu befürchten sind. Das anwendungsbereite Mittel wird bei solchen Systemen vom Verbraucher direkt vor der Anwendung durch Vermischen der Komponenten hergestellt. Im Falle eines oxidativen Färbemittels, bei dem die Oxidationsfarbstoffvorprodukte zunächst getrennt von einer Oxidationsmittelzubereitung, enthaltend bevorzugt Wasserstoffperoxid, vorliegen, ist dieses Vorgehen besonders bevorzugt.

Eine weitere Ausführungsform der vorliegenden Erfindung ist daher eine Mehrkomponenten-Verpackungseinheit ("Kit-of-parts") zum Färben und/oder Aufhellen keratinischer Fasern, wobei die Mehrkomponenten Verpackungseinheit einen ersten separat verpackten Container (A) enthält, der mindestens ein Mittel gemäß des ersten Erfindungsgegenstands enthält, und mindestens einen zweiten separat verpackten Container (B) enthält, der mindestens ein Mittel enthaltend Wasserstoffperoxid, enthält.

Unter Container der Mehrkomponenten-Verpackungseinheit wird im Rahmen der vorliegenden Erfindung eine Umhüllung verstanden, die in Form einer gegebenenfalls wieder verschließbaren Flasche, einer Tube, einer Dose, eines Tütchens, eines Sachets oder ähnlichen Umhüllungen vorliegt. Dem Umhüllungsmaterial sind erfindungsgemäß keine Grenzen gesetzt. Bevorzugt handelt es sich jedoch dabei um Umhüllungen aus Glas oder Kunststoff.

Weiterhin kann es erfindungsgemäß besonders vorteilhaft sein, wenn das genannte Kit-of-Parts mindestens ein weiteres Haarbehandlungsmittel in einem zusätzlichen Container enthält, insbesondere eine Konditioniermittelzubereitung oder ein Blondierpulver mit Peroxodisulfaten. Darüber hinaus kann die Verpackungseinheit Applikationshilfen, wie Kämme, Bürsten, Applicetten oder Pinsel, persönliche Schutzkleidung, insbesondere Ein-Weg-Handschuhe, sowie gegebenenfalls eine Gebrauchsanleitung umfassen. Unter einer Applicette wird ein breiter Pinsel verstanden, an dessen Stielende sich eine Spitze befindet, die das Abteilen von Faserbündeln bzw. Strähnchen aus der Gesamtmenge der Fasern erlaubt und vereinfacht.

In einer Ausführungsform des zweiten Erfindungsgegenstands enthält die Zubereitung des Containers (A) als farbverändernde Verbindung mindestens ein Oxidationsfarbstoffvorprodukt. Bezüglich weiterer bevorzugter Ausführungsformen der Mehrkomponentenverpackungseinheit (Kit-of-Parts) gilt mutatis mutandis das zu den erfindungsgemäßen Mitteln Gesagte.

Die Mittel des ersten Erfindungsgegenstandes können zum Färben und/oder Aufhellen von menschlichen Haaren verwendet werden.

Die Mittel des ersten Erfindungsgegenstands können in Verfahren zum Färben und/oder Aufhellen menschlicher Haare genutzt werden. Dabei wird ein Mittel des ersten Erfindungsgegenstands auf das Haar aufgetragen und für einen Zeitraum von 3 bis 45 Minuten, bevorzugt 5 bis 30 Minuten im Haar belassen. Anschließend wird das Haar mit Wasser und/oder einem handelsüblichen Shampoo gespült. Gewünschtenfalls kann das Haar anschließend mit einem weiteren Mittel behandelt werden und danach erneut mit Wasser und/oder einem handelsüblichen Shampoo gespült werden.

Die Auftragungs- und die Einwirktemperatur der Färbe- und/oder Aufhellzubereitung beträgt Raumtemperatur

bis 45°C. Gegebenenfalls kann die Wirkung der Färbe- und/oder Aufhellzubereitung durch externe Wärmezufuhr, wie beispielsweise mittels einer Wärmehaube, verstärkt werden. Die bevorzugte Einwirkdauer der Färbe- und/oder Aufhellzubereitung auf die keratinische Faser beträgt 3 bis 45 min, bevorzugt 5 bis 30 min. Nach Beendigung der Einwirkdauer wird das verbliebene Färbe- und/oder Aufhellmittel aus den keratinischen Fasern mit Hilfe einer Reinigungszubereitung oder Wasser ausgewaschen. Gegebenenfalls wird der Vorgang mit einem weiteren Mittel wiederholt. Nach dem Auswaschen werden die keratinischen Fasern gegebenenfalls mit einem Handtuch oder einem Heißluftgebläse getrocknet. Die Auftragung der Färbe- und/oder Aufhellzubereitung erfolgt üblicherweise mit der Hand durch den Anwender. Bevorzugt wird dabei persönliche Schutzkleidung getragen, insbesondere geeignete Schutzhandschuhe, beispielsweise aus Kunststoff oder Latex zur einmaligen Benutzung (Einweghandschuhe) sowie gegebenenfalls eine Schürze. Es ist aber auch möglich, die Färbe- und/oder Aufhellmittel mit einer Applikationshilfe auf die keratinischen Fasern aufzutragen.

In einer besonderen Ausführungsform dieses Verfahrens wird das anwendungsbereite Mittel zum Färben und/oder Aufhellen von keratinischen Fasern durch Vermischen der getrennt verpackten Mittel der Mehrkomponenten-Vverpackungseinheit des zweiten Erfindungsgegenstands hergestellt, auf die keratinischen Fasern aufgetragen, für eine bestimmte Einwirkzeit im Haar belassen und das Haar anschließend mit Wasser und/oder einem handelsüblichen Shampoo ausgespült.

Bezüglich weiterer bevorzugter Ausführungsformen der erfindungsgemäßen Verfahren und Verwendungen gilt mutatis mutandis das zu den erfindungsgemäßen Mitteln Gesagte.

Beispiele:

Die Mengenangaben verstehen sich, soweit nichts anderes vermerkt ist, jeweils in Gewichtsprozent.

### 1.1 Färbecremes

Es wurden die nachfolgenden Färbecremes hergestellt. Bei V1 und V2 handelt es sich um nicht erfindungsgemäße Vergleichsbeispiele, E1 und E2 sind die entsprechenden erfindungsgemäßen Rezepturen.

| Beschreibung | V1 | E1 | V2 | E2 |
|---|---|---|---|---|
| Carbomer | 1,1 | 1,1 | 2,0 | 2,0 |
| Ascorbinsäure | 0,2 | 0,2 | 0,2 | 0,2 |
| Emulgade F | 2,5 | 2,5 | 2,5 | 2,5 |
| Disodium Cocoamphodicacetate | 4,2 | 4,2 | 4,2 | 4,2 |
| Monoethanolamin | 5,0 | 5,0 | 5,0 | 5,0 |
| Dow Corning 193 C Fluid | 1,0 | --- | 1,0 | --- |
| Natriumsulfit | 0,2 | 0,2 | 0,2 | 0,2 |
| p-Toluylendiamin, Sulfat | 0,95 | 0,95 | 2,17 | 2,17 |
| 1,5-Dihydroxynaphthalin | 0,04 | 0,04 | 0,03 | 0,03 |
| Resorcin | 0,07 | 0,07 | 0,52 | 0,52 |
| 4-Chlorresorcin | 0,10 | 0,10 | 0,59 | 0,59 |
| 2-Methylresorcin | 0,30 | 0,30 | --- | --- |
| 3-Amino-2-methylamino-6-methoxypyridin | 0,08 | 0,08 | 0,08 | 0,08 |
| p-Amino-o-cresol | --- | --- | 0,05 | 0,05 |
| HEDP 60 % | 0,2 | 0,2 | 0,2 | 0,2 |
| Taurin | 2,0 | 2,0 | 2,0 | 2,0 |
| Coffein | 0,5 | 0,5 | 0,5 | 0,5 |
| Schwefelsäure 20 %ig | 0,21 | 0,21 | --- | --- |
| Natriumsilikat 40/42 | 0,5 | 0,5 | 0,5 | 0,5 |
| **Silfsoft AX** | --- | **1,0** | --- | **1,0** |

(fortgesetzt)

| Beschreibung | V1 | E1 | V2 | E2 |
|---|---|---|---|---|
| Wasser | ad 100 | ad 100 | ad 100 | ad 100 |

Eingesetzte Rohstoffe:

Carbomer (Polyacrylsäure)

Emulgade F (INCI-Name: CETEARYL ALCOHOL, PEG-40 CASTOR OIL, SODIUM CETEARYL SULFATE [BASF])
Dow Corning 193 C Fluid (INCI-Name: PEG-12 Dimethicone [Dow Corning])
HEDP (Hydroxyethane-1,1-diphosphonic acid,1-Etidronic acid)
Silsoft AX (INCI-Name: Bis-Cetearyl Amodimethicone [Momentive])

**1.2 Oxidationsmittelzubereitung**

Die zuvor hergestellten Färbecremes wurden direkt vor der Anwendung im Verhältnis 1:1 mit nachfolgender Oxidationsmittelzubereitung vermischt.

| Beschreibung | |
|---|---|
| EDTA, Dinatriumsalz | 0,15 |
| Dinatriumpyrophosphat | 0,30 |
| Natriumbenzoat | 0,04 |
| Emulgade F | 2,1 |
| Wasserstoffperoxid 50 % | 12,0 |
| Phosphorsäure | 0,04 |
| Wasser | ad 100 |

**1.3 Anwendung**

Die auf diese Weise hergestellten, anwendungsbereiten Färbeformulierungen wurden auf Strähnen Menschenhaares (Kerling) aufgetragen (Anwendungsverhältnis = 4 Gewichtsteile Creme : 1 Gewichtsteil Haar) und dort für 30 min bei 32 °C belassen. Nach Beendigung der Einwirkzeit wurde das Haar 2 Minuten lang mit Leitungswasser ausgespült (Wassertemperatur 32 °C). Nach dem Trocknen wurden intensiv gefärbte Haarsträhnen erhalten. Im Vergleich zu den nicht erfindungsgemäßen Beispielen besaßen die mit den erfindungsgemäßen Rezepturen gefärbten Haarsträhnen eine verbesserte Kämmbarkeit.

**1.4 Bestimmung des Haarglanzes**

Haartressen von 4 cm Breite und 15 cm Länge (Kerling, natural European Hair 6/0) wurden mit einer 0,3 %igen Texapon-Lösung (pH 6-7, INCI-Name: SODIUM LAURETH SULFATE) vorgereinigt und getrocknet. Diese Strähne wurden in der einer Glanzapparatur vermessen.

Es wurde die folgende Cremeformulierung hergestellt:

| Beschreibung | |
|---|---|
| Lanette D | 8,1 |
| Lorol C12-C18 techn. | 2,8 |
| Texapon NSO UP | 10,0 |
| Plantapon LGC Sorb | 5,0 |

(fortgesetzt)

| Beschreibung | |
|---|---|
| Eumulgin B1 | 0,25 |
| Ceteareth-20 | 0,25 |
| Iso-Stearinsäure | 2,0 |
| Myristic Acid | 0,5 |
| Natriumhydroxid 50 Gew.-% | 1,2 |
| Natriumsulfit (wasserfrei) | 0,5 |
| Ascorbinsäure | 0,3 |
| Ammoniumsulfat | 0,4 |
| **Silsoft AX** | **4,0** |
| Natriumsilikat 40/42 | 0,5 |
| HEDP 60 % | 0,2 |
| Ammoniak 25 % | 6,3 |
| Wasser | ad 100 |

Eingesetzte Rohstoffe:

Lanette D (INCI-Name: CETEARYL ALCOHOL)

Lorol C12-C18 techn.: C12-C18 fatty alcohols

Texapon NSO UP (INCI-Name: SODIUM LAURETH SULFATE), 27 %ig

Plantapon LGC Sorb (INCI-Name: LAURYL GLUCOSE CARBOXYLATE, LAURYL GLUCOSIDE)

Eumulgin B1 (INCI-Name: CETEARETH-12)
HEDP (Hydroxyethane-1,1-diphosphonic acid,1-Etidronic acid)

Vor der Anwendung wurde die obige Cremeformulierung im Verhältnis 1:1 mit der nachfolgenden Oxidationsmittelzubereitung vermischt:

| Beschreibung | |
|---|---|
| Ammoniak 25 % | 0,62 |
| Dipicolinsäure | 0,1 |
| Dinatriumpyrophosphat | 0,03 |
| HEDP 60 % | 1,5 |
| Texapon NSO UP | 2,0 |
| Dow Corning 110 A | 0,07 |
| Aculyn 33 A | 12,0 |
| Wasserstoffperoxid 50 % | 12,0 |

Eingesetzte Rohstoffe:

Dow Corning 110 A (INCI-Name: DIMETHICONE), 10 %ig
Aculyn 33 A (INCI-Name: ACRYLATES COPOLYMER), 27-29 %ig

Das auf diese Weise hergestellte anwendungsbereite Haarbehandlungsmittel wurde auf die Haartressen aufgebracht (4 g Anwendungsmischung pro g Haar) und für 30 min bei 32 °C einwirken gelassen. Danach wurden die Tressen mit Leitungswasser ausgespült und getrocknet. Die getrockneten Haarsträhnen wurden ein zweites Mal in einer Glanzapparatur vermessen.

Bei der Glanzapparatur handelt es sich um eine mit einer lichtabsorbierenden Auskleidung versehene Glanzkammer. Die zu vermessenden Haarsträhnen wurden auf einen Zylinder gespannt und in der Mitte der Glanzkammer positioniert. Oberhalb des Zylinders befindet sich als Beleuchtungsquelle eine stabförmige Gasentladungslampe, welche die auf dem Zylinder aufgespannte Haarsträhne durch eine kleine Blende beleuchtet. Mit einer Digitalkamera wurde die Glanzkurve jeder Strähne vermessen und bildanalytisch ausgewertet. Anschließend wurde der Glanzwert der Strähnen (L) nach der Formel nach Reich und Robbins (1) berechnet:

$$L_{Reich/Robbins} = \frac{S}{D * W_{\frac{1}{2}}}$$

L: Glanz
S: gerichtete Reflexion
D: diffuse Reflexion
W½: Halbwertsbreite der Glanzkurve

(1) C. Reich and C.C. Robbins, J. Soc. Cosmet. Chem. **44,** 221-234 (1993)

Die Veränderung des Glanzes wurde durch Vergleich der vor und nach der Applikation des erfindungsgemäßen Mittels gemessenen Glanzwerte bewertet. Mittels statistischer Verfahren wurde berechnet, ob die Änderung des Glanzes signifikant ist.

Durch Applizierung der erfindungsgemäßen Formulierung wurde der Glanz der Haarsträhne statistisch signifikant um 33 % vermindert. Analoge Ergebnisse wurden auch mit farbstoffhaltigen Formulierungen erhalten.

## Patentansprüche

1. Mittel zum Färben und/oder Aufhellen von Haaren mit einem pH-Wert im Bereich von 8,0 bis 12,0, **dadurch gekennzeichnet, dass** es in einem kosmetisch geeigneten Träger

a. 0,01 bis 15 Gew.-%, bezogen auf das Gesamtgewicht des Mittels, mindestens eines Alkalisierungsmittels aus der Gruppe der Alkanolamine, der basischen Aminosäuren oder deren Gemische und
b. mindestens eine Verbindung der Formel (I)

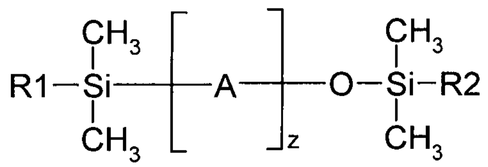
(I)

mit

A = 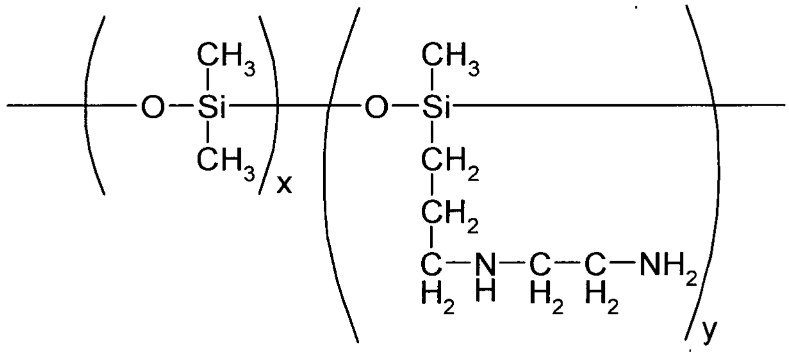

worin

i. x und y unabhängig von einander für Werte zwischen 1 und 100 stehen und
ii. z für Werte zwischen 1 und 100 steht, wobei, falls $z \geq 2$ ist, die jeweiligen Werte x und y in einem Strukturelement A jeweils unabhängig von vorangehenden Strukturelementen A gewählt werden können und
iii. R1 für $H_3C\text{-}(CH_2)_{15}\text{-}$ oder $H_3C\text{-}(CH_2)_{17}\text{-}$ und R2 für $H_3C\text{-}(CH_2)_{15}\text{-}$ oder $H_3C\text{-}(CH_2)_{17}\text{-}$ steht, und

c. mindestens ein Oxidationsfarbstoffvorprodukt vom Entwickler- und/oder Kupplertyp enthält.

2. Mittel zum Färben und/oder Aufhellen von Haaren **dadurch gekennzeichnet, dass** es in einem kosmetisch geeigneten Träger

a. mindestens ein Oxidationsfarbstoffvorprodukt
b. 0,01 bis 15 Gew.-%, bezogen auf das Gesamtgewicht des Mittels, mindestens eines Alkalisierungsmittels aus der Gruppe der Alkanolamine, der basischen Aminosäuren oder deren Gemische, und
c. mindestens eine Verbindung der Formel (I)

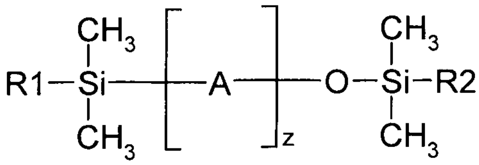
(I)

mit

A = 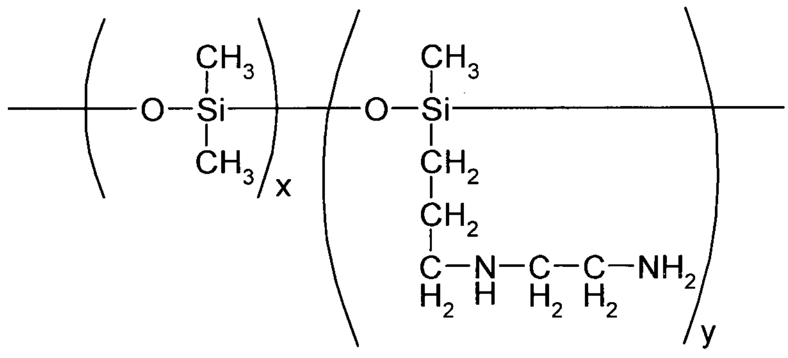

worin

i. x und y unabhängig von einander für Werte zwischen 1 und 100 stehen und
ii. z für Werte zwischen 1 und 100 steht, wobei, falls $z \geq 2$ ist, die jeweiligen Werte x und y in einem Strukturelement A jeweils unabhängig von vorangehenden Strukturelementen A gewählt werden können

und
iii. R1 für $H_3C-(CH_2)_{15}-$ oder $H_3C-(CH_2)_{17}-$ und R2 für $H_3C-(CH_2)_{15}-$ oder $H_3C-(CH_2)_{17}-$ steht, enthält, mit der Maßgabe, dass das Mittel nicht gleichzeitig Ammoniak und Kaliumhydroxid enthält.

3. Mittel nach einem der Ansprüche 1 oder 2, **dadurch gekennzeichnet, dass** es 0,005 bis 5 Gew.-%, bevorzugt 0,1 bis 4,5 Gew.-%, besonders bevorzugt 1 bis 3 Gew.-% und insbesondere bevorzugt 1,5 bis 2,5 Gew.-% der Verbindung/en der Formel (I), bezogen auf das Gesamtgewicht des Mittels, enthält.

4. Mittel nach einem der Ansprüche 1 bis 3, **dadurch gekennzeichnet, dass** es weniger als 1 Gew.-%, vorzugsweise weniger als 0,1 Gew.-%, besonders bevorzugt weniger als 0,01 Gew.-% und insbesondere bevorzugt weniger als 0,001 Gew.-% Ammoniak, bezogen auf das Gesamtgewicht des Mittels, enthält.

5. Mittel nach einem der Ansprüche 1 bis 4, **dadurch gekennzeichnet, dass** es, bezogen auf das Gesamtgewicht des Mittels, 0,1 bis 10 Gew.-%, besonders bevorzugt 1 bis 8 Gew.-% und insbesondere bevorzugt 3 bis 7 Gew.-% mindestens eines Alkalisierungsmittels aus der Gruppe der Alkanolamine, der basischen Aminosäuren oder deren Gemische enthält.

6. Mittel nach einem der Ansprüche 1 bis 5, **dadurch gekennzeichnet, dass** es ein Oxidationsfarbstoffvorprodukt vom Entwickler und/oder Kupplertyp in einem Gewichtsanteil von 0,001 bis 10 Gew.-%, bevorzugt 0,01 bis 8 Gew.-%, besonders bevorzugt 0,1 bis 5 Gew.-% und insbesondere bevorzugt 0,5 bis 3 Gew.-%, bezogen auf das Gesamtgewicht des Mittels, enthält.

7. Mittel nach einem der Ansprüche 1 bis 6, **dadurch gekennzeichnet, dass** es einen direktziehenden Farbstoff in einem Gewichtsanteil von 0,001 bis 10 Gew.-%, bevorzugt 0,01 bis 8 Gew.-%, besonders bevorzugt 0,1 bis 5 Gew.-% und insbesondere bevorzugt 0,5 bis 3 Gew.-%, bezogen auf das Gesamtgewicht des Mittels, enthält.

8. Mittel nach einem der Ansprüche 1 bis 7, **dadurch gekennzeichnet, dass** es zusätzlich einen Wirkstoff ausgewählt aus Panthenol, Pantothensäure, Pantolacton, Allantoin, Bisabolol, Carnitin, Coffein, Theophyllin, Theobromin und/oder Taurin, bevorzugt ausgewählt aus Coffein, Theophyllin, Theobromin und/oder Taurin, enthält.

9. Anwendungsbereites Mittel nach einem der Ansprüche 1 bis 8, **dadurch gekennzeichnet, dass** es zusätzlich als Oxidationsmittel Wasserstoffperoxid und/oder eines seiner festen Anlagerungsprodukte an organische oder anorganische Verbindungen in einem Gewichtsanteil von 0,5 bis 12 Gew.-%, vorzugsweise 2 bis 10 Gew.-% und insbesondere bevorzugt 3 bis 6 Gew.-% (berechnet als 100 %-iges $H_2O_2$), bezogen auf das Gesamtgewicht des Mittels, enthält.

10. Verfahren zum Färben und/oder Aufhellen von Haaren bei dem

a. ein Mittel M1 zum Färben und/oder Aufhellen auf der Faser zur Anwendung kommt, wobei gewünschtenfalls dem Mittel M1 vor der Anwendung ein weiteres Mittel M2 zugegeben wird und
b. diese Mittel nach einer Zeit von 5 bis 45 Minuten von der Faser abgespült werden,

**dadurch gekennzeichnet, dass** das Mittel M1 ein Mittel gemäß einem der Ansprüche 2 bis 9 und das Mittel M2 ein Mittel enthaltend Wasserstoffperoxid ist.

## Claims

1. An agent for dyeing and/or lightening hair, having a pH in the range of from 8.0 to 12.0, **characterized in that** it contains, in a cosmetically suitable carrier,

a. from 0.01 to 15 wt.%, based on the total weight of the agent, of at least one alkalizing agent from the group of alkanolamines, basic amino acids or mixtures thereof and
b. at least one compound of formula (I)

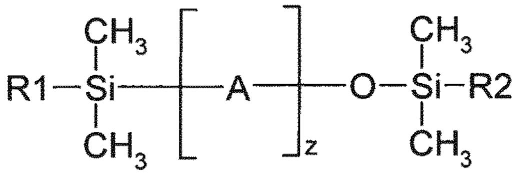 (I)

with

A = 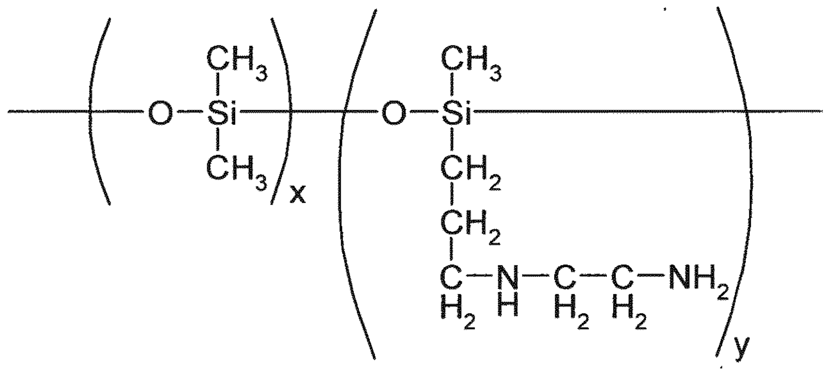

where

i. x and y represent, independently of one another, values between 1 and 100 and
ii. z represents values between 1 and 100, where, if $z \geq 2$, the respective values x and y in a structural element A may each be selected independently of preceding structural elements A
and
iii. R1 represents $H_3C\text{-}(CH_2)_{15}\text{-}$ or $H_3C\text{-}(CH_2)_{17}\text{-}$ and R2 represents $H_3C\text{-}(CH_2)_{15}\text{-}$ or $H_3C\text{-}(CH_2)_{17}\text{-}$, and

c. at least one developer-type and/or coupler-type oxidation dye precursor.

**2.** The agent for dyeing and/or lightening hair, **characterized in that** it contains, in a cosmetically suitable carrier,

a. at least one oxidation dye precursor
b. from 0.01 to 15 wt.%, based on the total weight of the agent, of at least one alkalizing agent from the group of alkanolamines, basic amino acids or mixtures thereof, and
c. at least one compound of formula (I)

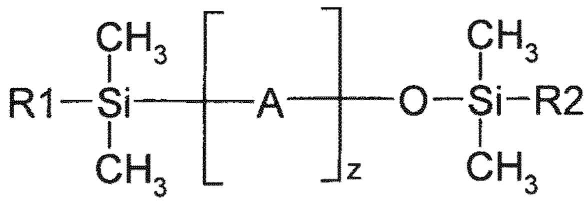 (I)

with

A = 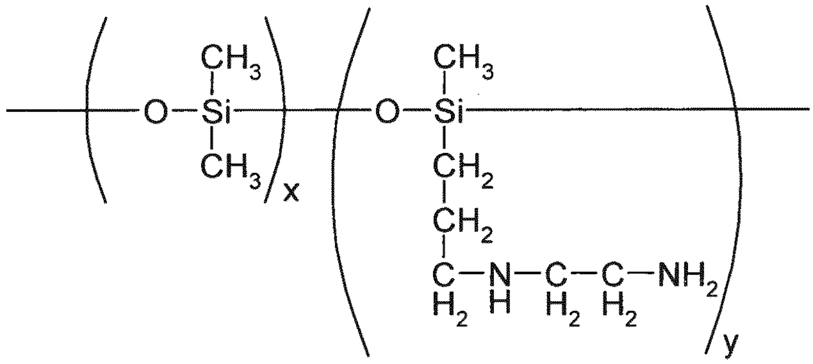

where

i. x and y represent, independently of one another, values between 1 and 100 and
ii. z represents values between 1 and 100, where, if z ≥ 2, the respective values x and y in a structural element A may each be selected independently of preceding structural elements A
and
iii. R1 represents $H_3C-(CH_2)_{15}$- or $H_3C-(CH_2)_{17}$- and R2 represents $H_3C-(CH_2)_{15}$- or $H_3C-(CH_2)_{17}$-,

with the proviso that the agent does not simultaneously contain ammonia and potassium hydroxide.

3. The agent according to one of claims 1 or 2, **characterized in that** it contains from 0.005 to 5 wt.%, preferably from 0.1 to 4.5 wt.%, particularly preferably from 1 to 3 wt.%, and more particularly preferably from 1.5 to 2.5 wt.%, of the compound(s) of formula (I), based on the total weight of the agent.

4. The agent according to one of claims 1 to 3, **characterized in that** it contains less than 1 wt.%, preferably less than 0.1 wt.%, particularly preferably less than 0.01 wt.%, and more particularly preferably less than 0.001 wt.%, of ammonia, based on the total weight of the agent.

5. The agent according to one of claims 1 to 4, **characterized in that** it contains, based on the total weight of the agent, from 0.1 to 10 wt.%, particularly preferably from 1 to 8 wt.%, and more particularly preferably from 3 to 7 wt.%, of at least one alkalizing agent from the group of alkanolamines, basic amino acids or mixtures thereof.

6. The agent according to one of claims 1 to 5, **characterized in that** it contains a developer-type and/or coupler-type oxidation dye precursor in a weight proportion of from 0.001 to 10 wt.%, preferably from 0.01 to 8 wt.%, particularly preferably from 0.1 to 5 wt.%, and more particularly preferably from 0.5 to 3 wt.%, based on the total weight of the agent.

7. The agent according to one of claims 1 to 6, **characterized in that** it contains a direct dye in a weight proportion of from 0.001 to 10 wt.%, preferably from 0.01 to 8 wt.%, particularly preferably from 0.1 to 5 wt.%, and more particularly preferably from 0.5 to 3 wt.%, based on the total weight of the agent.

8. The agent according to one of claims 1 to 7, **characterized in that** it additionally contains an active ingredient selected from panthenol, pantothenic acid, pantolactone, allantoin, bisabolol, carnitine, caffeine, theophylline, theobromine and/or taurine, preferably selected from caffeine, theophylline, theobromine and/or taurine.

9. The ready-to-apply agent according to one of claims 1 to 8, **characterized in that** it additionally contains hydrogen peroxide and/or one of the solid addition products thereof to organic or inorganic compounds as an oxidizing agent in a weight proportion of from 0.5 to 12 wt.%, preferably from 2 to 10 wt.%, and particularly preferably from 3 to 6 wt.% (calculated as 100% $H_2O_2$), based on the total weight of the agent.

10. A method for dyeing and/or lightening hair, in which

a. a dyeing and/or lightening agent M1 is used on the fiber, an additional agent M2 being added, if desired, to the agent M1 before said agent is used, and
b. said agent is rinsed off the fiber after a period of 5 to 45 minutes,

**characterized in that** the agent M1 is an agent according to one of claims 2 to 9 and the agent M2 is an agent containing hydrogen peroxide.

## Revendications

1. Agent de coloration et/ou d'éclaircissement de cheveux ayant une valeur pH compris dans une plage allant de 8,0 à 12,0, **caractérisé en ce qu'**il contient dans un support cosmétiquement approprié

    a. de 0,01 à 15 % en poids, rapporté au poids total de l'agent, d'au moins un agent d'alcalinisation issu du groupe des alcanolamines, des acides aminés basiques ou des mélanges de ces derniers et
    b. au moins un composé de la formule (I)

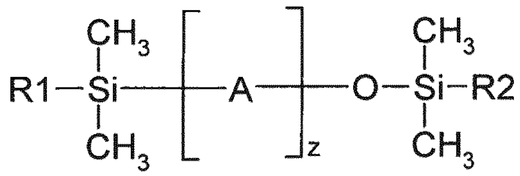

(I)

avec

A =

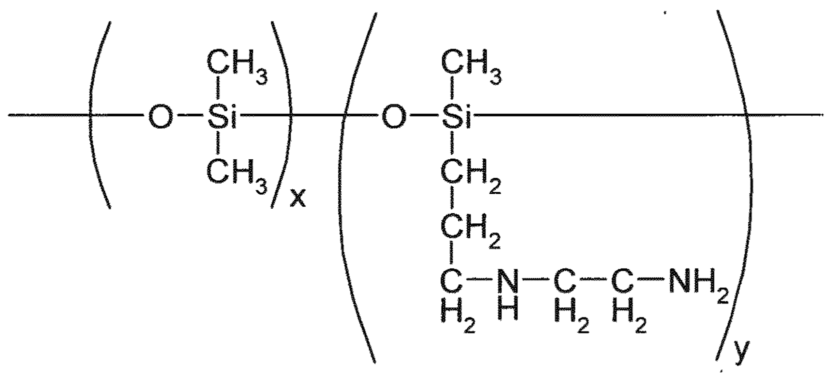

dans laquelle

i. x et y représentent indépendamment l'un de l'autre des valeurs comprises entre 1 et 100 et
ii. z représente des valeurs comprises entre 1 et 100, dans lesquelles, si z ≥ 2, les valeurs x et y respectives peuvent être choisies dans un élément de structure A indépendamment des éléments de structure A précédents
et
iii. R1 représente $H_3C-(CH_2)_{15}-$ ou $H_3C-(CH_2)_{17}-$ et R2 représente $H_3C-(CH_2)_{15}-$ ou $H_3C-(CH_2)_{17}$, et

c. au moins un précurseur de colorant par oxydation de type développeur et/ou de type coupleur.

2. Agent de coloration et/ou d'éclaircissement de cheveux, **caractérisé en ce qu'**il contient dans un support cosmétiquement approprié

    a. au moins un précurseur de colorant par oxydation
    b. de 0,01 à 15 % en poids, rapporté au poids total de l'agent, d'au moins un agent d'alcalinisation issu du groupe des alcanolamines, des acides aminés basiques ou des mélanges de ces derniers et
    c. au moins un composé de la formule (I)

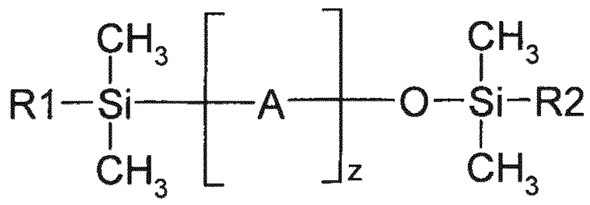

(I)

avec

A = 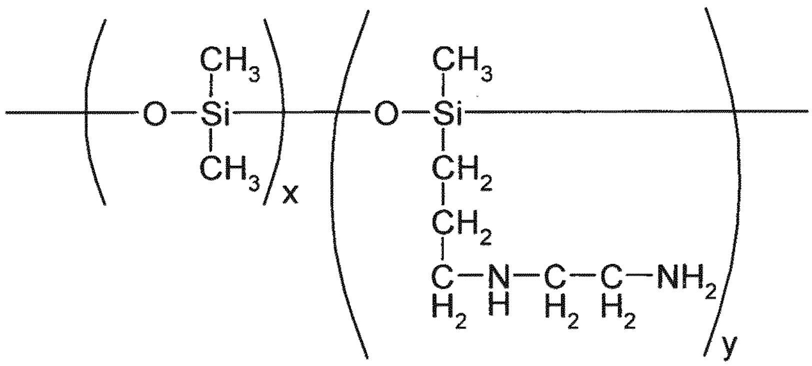

dans laquelle

i. x et y représentent indépendamment l'un de l'autre des valeurs comprises entre 1 et 100 et
ii. z représente des valeurs comprises entre 1 et 100, dans lesquelles, si $z \geq 2$, les valeurs x et y respectives peuvent être choisies dans un élément de structure A indépendamment des éléments de structure A précédents
et
iii. R1 représente $H_3C-(CH_2)_{15}-$ ou $H_3C-(CH_2)_{17}-$ et R2 représente $H_3C-(CH_2)_{15}-$ ou $H_3C-(CH_2)_{17}$, et

à la condition que l'agent ne contienne pas simultanément de l'ammoniac et de l'hydroxyde de potassium.

3. Agent selon l'une des revendications 1 ou 2, **caractérisé en ce qu'**il contient de 0,005 à 5 % en poids, de manière préférée de 0,1 à 4,5 % en poids, de manière particulièrement préférée de 1 à 3 % en poids et en particulier de manière préférée de 1,5 à 2,5 % en poids des composés de la formule (I), rapporté au poids total de l'agent.

4. Agent selon l'une des revendications 1 à 3, **caractérisé en ce qu'**il contient moins de 1 % en poids, de préférence moins de 0,1 % en poids, de manière particulièrement préférée moins de 0,01 % en poids et en particulier de manière préférée moins de 0,001 % en poids en ammoniac, rapporté au poids total de l'agent.

5. Agent selon l'une des revendications 1 à 4, **caractérisé en ce qu'**il contient, rapporté au poids total de l'agent, de 0,1 à 10 % en poids, de manière particulièrement préférée de 1 à 8 % en poids et en particulier de manière préférée de 3 à 7 % en poids d'au moins un agent d'alcalinisation issu du groupe des alcanolamines, des acides aminés basiques, ou des combinaisons de ces derniers.

6. Agent selon l'une des revendications 1 à 5, **caractérisé en ce qu'**il contient un précurseur de colorant par oxydation de type développeur et/ou de type coupleur dans une proportion en poids de 0,001 à 10 % en poids, de manière préférée de 0,01 à 8 % en poids, de manière particulièrement préférée de 0,1 à 5 % en poids et en particulier de manière préférée de 0,5 à 3 % en poids, rapporté au poids total de l'agent.

7. Agent selon l'une des revendications 1 à 6, **caractérisé en ce qu'**il contient un colorant à action directe dans une proportion en poids de 0,001 à 10 % en poids, de manière préférée de 0,01 à 8 % en poids, de manière particulièrement préférée de 0,1 à 5 % en poids et en particulier de manière préférée de 0,5 à 3 % en poids, rapporté au poids total de l'agent.

8. Agent selon l'une des revendications 1 à 7, **caractérisé en ce qu'**il contient de plus un principe actif choisi parmi

le panthénol, l'acide pantothénique, le pantolactone, l'allantoïne, le bisabolol, la carnitine, la caféine, la théophylline, la théobromine et/ou la taurine, de manière préférée parmi la caféine, la théophylline, la théobromine et/ou la taurine.

**9.** Agent prêt à l'emploi selon l'une des revendications 1 à 8, **caractérisé en ce qu'**il contient en plus, en tant qu'agent d'oxydation, du peroxyde d'hydrogène et/ou un de ses produits d'addition solide sur des composés organiques ou inorganiques dans une proportion en poids allant de 0,5 à 12 % en poids, de préférence de 2 à 10 % en poids et en particulier de manière préférée de 3 à 6 % en poids (calculé une teneur de 100 % en $H_2O_2$), rapporté au poids total de l'agent.

**10.** Procédé de coloration et/ou d'éclaircissement de cheveux lors duquel

a. un agent M1 de coloration et/ou d'éclaircissement est utilisé sur les fibres, auquel un autre agent M2 est si nécessaire ajouté à l'agent M1 avant l'application et
b. ces agents sont rincés des fibres après une durée de 5 à 45 minutes,

**caractérisé en ce que** l'agent M1 est un agent selon l'une des revendications 2 à 9 et l'agent M2 est un agent contenant du peroxyde d'hydrogène.